# EUROPEAN PATENT APPLICATION

(11) **EP 4 467 652 A1**
(43) Date of publication of application: **27.11.2024**
(21) Application number: 23743530.0
(22) Date of filing: 20.01.2023
(51) Int. Cl.: C12N 15/77, C12P 7/18

(54) **MICROORGANISM FOR PRODUCING 1,4-BUTANEDIOL AND METHOD FOR PRODUCING 1,4-BUTANEDIOL BY USING SAME**

(30) Priority: 21.01.2022 KR 20220009016
(71) Applicant: Daesang Corporation, Seoul 02586 (KR)
(72) Inventor: YANG, Young Lyeol, Seoul 07789 (KR); PARK, Si Jae, Goyang-si, Gyeonggi-do 10234 (KR); SON, Jin A, Seoul 04717 (KR); JEONG, Seon A, Seoul 03765 (KR); SOHN, Yu Jung, Seoul 06587 (KR); LEE, Jae Hun, Seoul 07789 (KR); KIM, Moon Jeong, Seoul 07789 (KR)
(74) Representative: Cabinet Nony
(86) International application number: PCT/KR2023/001023
(87) International publication number: WO 2023/140687

(57) **Abstract**

The present invention relates to a microorganism that produces 1,4-butanediol and a method of producing 1,4-butanediol using same. The microorganism according to the present invention is able to efficiently produce 1,4-butanediol using ornithine as a carbon source, unlike naturally occurring microorganisms.

## Description

### Technical Field

The present invention relates to a microorganism that produces 1,4-butanediol and a method of producing 1,4-butanediol using the microorganism.

### Background Art

1,4-Butanediol is used as a solvent, polymer intermediate, and fine chemical intermediate throughout the chemical industry. 1,4-Butanediol is mainly produced through a process of reacting acetylene with formaldehyde, followed by hydrogenation. In addition, 1,4-butanediol can also be produced from maleic anhydride and propylene oxide. However, there are problems such as the generation of global warming gases and waste due to the use of fossil fuels, as well as disruptions in the supply of raw materials and increased production costs due to unstable international oil prices.

To overcome these problems of the chemical production processes, low-cost and environmentally friendly processes of biologically producing 1,4-butanediol using biomass as a raw material have recently been developed. Generally, 1,4-butanediol is produced via α-ketoglutarate or succinyl-CoA in microbial metabolic process. However, in the process of producing 1,4-butanediol using a microorganism, there are limitations in that unnecessary byproducts are produced in addition to 1,4-butanediol in the microbial metabolic process and the production yield is relatively low. Therefore, to overcome these limitations, efforts have been continuously made to develop microorganisms, which are capable of producing 1,4-butanediol in high yields, using genetic engineering techniques.

### [Prior Art Documents]

### [Patent Documents]

(Patent Document 1) European Patent No. 3050970
(Patent Document 2) European Patent No. 2782893

### DISCLOSURE

### Technical Problem

An object of the present invention is to provide a microorganism that produces 1,4-butanediol.

Another object of the present invention is to provide a method of producing 1,4-butanediol using the microorganism.

### Technical Solution

In the previously known 1,4-butanediol synthesis pathway in microorganisms, pyruvate produced from glucose by glycolysis is oxidized to acetyl-CoA which is then synthesized into α-ketoglutarate and succinyl-CoA in the TCA cycle. Here, 4-hydroxybutyric acid is produced through decarboxylation of α-ketoglutarate or reduction of succinyl-CoA, and 1,4-butanediol is finally produced therefrom.

However, the present inventors have established a novel 1,4-butanediol production pathway that uses ornithine instead of α-ketoglutarate or succinyl-CoA as a starting material, and have found that a microorganism with this production pathway is able to efficiently produce 1,4-butanediol by high enzyme activity, thereby completing the present invention.

One aspect of the present invention provides a microorganism that produces 1,4-butanediol from ornithine.

The term "microorganism" as used in the present invention refers to prokaryotic cells or eukaryotes, and includes bacteria, yeast, fungi, etc.

The phrase "microorganism that produces 1,4-butanediol" as used in the present invention refers to a microorganism containing enzymes involved in the 1,4-butanediol production pathway or genes encoding the enzymes. The pathway of producing 1,4-butanediol from ornithine in the present invention is a production pathway that has not been identified in microorganisms known to date. Therefore, in the present invention, the microorganism that produces 1,4-butanediol (hereinafter referred to as "1,4-butanediol-producing microorganism") refers to a modified microorganism obtained by modifying an endogenous gene sequence (nucleic acid deletion, addition, substitution, etc.), inserting a foreign gene into the genome, and/or introducing a plasmid (vector) containing a foreign gene, to introduce a gene encoding an enzyme involved in the ornithine-1,4-butanediol production pathway. In the present invention, the term "modified microorganism" may be used interchangeably with the term "mutant microorganism", "mutant strain", "recombinant microorganism", "recombinant strain", "genetically modified microorganism", or "genetically modified strain". In contrast, the term "microorganism before modification", "strain before modification", "non-mutated microorganism", "non-mutated strain", or "parent strain" refers to a microorganism before the trait thereof is changed by genetic mutation due to natural or artificial factors, for example, a microorganism that does not/cannot produce 1,4-butanediol due to the lack of enzymes that acts in the ornithine-1,4-butanediol production pathway.

According to one embodiment of the present invention, the 1,4-butanediol-producing microorganism may have an enzymatic reaction that converts ornithine to 4-aminobutanamide, an enzymatic reaction that converts 4-aminobutanamide to 4-aminobutyric acid, an enzymatic reaction that converts 4-aminobutyric acid to succinate semialdehyde, an enzymatic reaction that converts succinate semialdehyde to 4-hydroxybutyric acid, and an enzymatic reaction that converts 4-hydroxybutyric acid to 1,4-butanediol.

According to one embodiment of the present invention, the enzyme that catalyzes the enzymatic reaction that converts ornithine to 4-aminobutanamide may be lysine 2-monooxygenase encoded by the davB gene.

According to one embodiment of the present invention, the enzyme that catalyzes the enzymatic reaction that converts 4-aminobutanamide to 4-aminobutyric acid may be 5-aminovaleramidase encoded by the davA gene.

According to one embodiment of the present invention, the enzyme that catalyzes the enzymatic reaction that converts the 4-aminobutyric acid to succinate semialdehyde may be gamma-aminobutyrate aminotransferase encoded by the gabT gene.

According to one embodiment of the present invention, the enzyme that catalyzes the enzymatic reaction that converts succinate semialdehyde to 4-hydroxybutyric acid may be alcohol dehydrogenase encoded by the yahK gene.

According to one embodiment of the present invention, the enzyme that catalyzes the enzymatic reaction that converts 4-hydroxybutyric acid to 1,4-butanediol may be an alcohol dehydrogenase encoded by the yahK gene or carboxylic acid reductase encoded by the car gene.

The carboxylic acid reductase may be activated by 4'-phosphopantetheinyl transferase encoded by the sfp gene.

The lysine 2-monooxygenase, 5-aminovaleramidase, gamma-aminobutyrate aminotransferase, alcohol dehydrogenase and carboxylic acid reductase contained in the 1,4-butanediol-producing microorganism are enzymes that are not present in the microorganism before modification. In addition, the 4'-phosphopantetheinyl transferase may or may not be present in the microorganism before modification.

According to one embodiment of the present invention, the enzymes may be obtained by introduction of foreign genes.

More specifically, the enzymes can catalyze the expression and enzymatic reaction of enzymes for producing 1,4-butanediol from ornithine, by artificially introducing genes selected from the group consisting of davB, davA, gabT, yahK, car and sfp present in common microorganisms or sequences having substantial identity thereto into a 1,4-butanediol-producing microorganism.

The term "substantial identity" as used in the present invention means that, when each gene sequence, i.e., a base sequence or nucleotide sequence, and any other nucleotide sequence are aligned to correspond to each other as much as possible and analyzed, the other nucleotide sequence has a sequence homology of at least 70%, at least 80%, at least 90%, or at least 98% with each nucleotide sequence.

According to one embodiment of the present invention, the genes davB, davA, gabT, yahK, car and sfp encoding the enzymes may be derived from microorganisms known in the art, and the type of microorganism is not particularly limited.

More specifically, the genes encoding the enzymes may be derived from one or more microorganisms selected from the group consisting of *Escherichia coli, Pseudomonas putida, Corynebacterium glutamicum, Mycobacterium abscessus, Bacillus subtilis, Acinetobacter baumannii, Azotobacter vinelandii, Chromohalobacter salexigens, Citrobacter koseri, Citrobacter youngae, Enterobacter cloacae, Marinobacter aquaeolei, Marinomonas mediterranea, Pantoea ananatis, Pseudoalteromonas haloplanktis, Ralstonia eutropha, Shewanella putrefaciens,* and *Thiobacillus denitrificans.*

For example, the davB gene may be represented by the nucleotide sequence of SEQ ID NO: 1, or may encode the amino acid sequence of SEQ ID NO: 2, without being limited thereto.

The davA gene may be represented by the nucleotide sequence of SEQ ID NO: 3, or may encode the amino acid sequence of SEQ ID NO: 4, without being limited thereto.

The gabT gene may be represented by the nucleotide sequence of SEQ ID NO: 5, or may encode the amino acid sequence of SEQ ID NO: 6, without being limited thereto.

The yahK gene may be represented by the nucleotide sequence of SEQ ID NO: 7, or may encode the amino acid sequence of SEQ ID NO: 8, without being limited thereto.

The car gene may be represented by the nucleotide sequence of SEQ ID NO: 9, or may encode the amino acid sequence of SEQ ID NO: 10, without being limited thereto.

The sfp gene may be represented by the nucleotide sequence of SEQ ID NO: 11, or may encode the amino acid sequence of SEQ ID NO: 12, without being limited thereto.

The 1,4-butanediol producing microorganism according to the present invention may be produced by introducing or transforming the davB, davA, gabT, yahK, car and sfp genes into a host cell through one or more vectors containing the genes all or individually.

The term "vector" as used in the present invention refers to any type of nucleic acid sequence carrier construct that is used as a means for delivering and expressing a gene of interest in a host cell. Unless otherwise specified, the term "vector" may refer to a construct allowing the carried nucleic acid sequence to be inserted into the genome of a host cell and expressed in the host cell, and/or expressed independently. The vector contains essential regulatory elements operably linked so that the inserted gene is expressed. Here, "operably linked" means that the gene of interest and regulatory sequences therefor are functionally linked to one another in a manner enabling gene expression. "Regulatory elements" includes a promoter for initiating transcription, any operator sequence for controlling transcription, a sequence encoding suitable mRNA ribosome binding sites, and a sequence for controlling termination of transcription and translation.

The vector that is used in the present invention is not particularly limited as long as it is replicable in a host cell, and any vector known in the art may be used. Examples of the vector may include natural or recombinant plasmids, cosmids, viruses, and bacteriophages. For example, phage vectors or cosmid vectors include, but are not limited to, pWE15, M13, AMBL3, AMBL4, λIXII, AASHII, λAPII, λt10, λt11, Charon4A, Charon21A, etc., and plasmid vectors include, but are not limited to, pBR-based, pUC-based, pBluescriptII-based, pGEM-based, pTZ-based, pCL-based and pET-based vectors.

The vector may typically be constructed as a vector for cloning or a vector for expression. The vector for expression may be a conventional vector that is used in the art to express foreign genes or proteins in plants, animals, or microorganisms, and may be constructed by various methods known in the art.

The recombinant vector may be constructed using a prokaryotic or eukaryotic cell as a host. For example, when the vector to be used is an expression vector and employs a eukaryotic cell as a host, it generally contains a strong promoter capable of initiating transcription (e.g., pLλ, promoter, CMV promoter, trp promoter, lac promoter, tac promoter, or T7 promoter), a ribosome binding site for initiating translation, and a transcription/translation termination sequence. When the vector employs a prokaryotic cell as a host, eukaryotic origins of replication that may be contained in the vector include, but are not limited to, an f1 replication origin, an SV40 replication origin, a pMB1 replication origin, an adeno replication origin, an AAV replication origin, and a BBV replication origin. In addition, a promoter derived from the genome of a mammalian cell (e.g., a metallothionein promoter) or a promoter derived from a mammalian virus (e.g., an adenovirus late promoter, a vaccinia virus 7.5K promoter, an SV40 promoter, a cytomegalovirus promoter, or a tk promoter of HSV) may be used, and the vector generally has a polyadenylation sequence as a transcription termination sequence.

The recombinant vector may contain a selection marker. The selection marker may be used to select a transformant (host cell) obtained by transformation with the vector. Since only cells expressing the selection marker may survive in the medium treated with the selection marker, the selection marker may select the transformed cells. Representative examples of the selection marker include, but are not limited to, kanamycin, streptomycin, and chloramphenicol.

A transformant may be produced by inserting the recombinant vector into a host cell, and the transformant may be obtained by introducing the recombinant vector into an appropriate host cell. As the host cell, any host cell known in the art may be used which is capable of stably and continuously cloning or expressing the expression vector.

When the vector is transformed into a prokaryotic cell to produce a recombinant microorganism, examples of the host cell that may be used include *E. coli* strains such as *E. coli* JM109, *E. coli* BL21, *E. coli* RR1, *E. coli* LE392, *E. coli* B, *E. coli* X 1776, *E. coli* W3110, and *E. coli* XL1-Blue; *Bacillus* sp. strains such as *Bacillus subtilis* and *Bacillus thuringiensis; Corynebacterium* sp. strains; and a variety of enteric bacteria and strains, such as *Salmonella typhimurium, Serratia marcescens* and *Pseudomonas* species.

When the vector is transformed into a eukaryotic cell to produce a recombinant microorganism, examples of the host cell that may be used include, but are not limited to, yeast (e.g., *Saccharomyces cerevisiae*), insect cells, plant cells, and animal cells, for example, Sp2/0, CHO K1, CHO DG44, PER.C6, W138, BHK, COS7, 293, HepG2, Huh7, 3T3, RIN, and MDCK cell lines.

The term "transformation" as used in the present invention refers to a phenomenon of artificially causing a genetic change by introducing exogenous DNA into a host cell, and the term "transformant" refers to a host cell into which exogenous DNA has been introduced and in which the expression of a gene of interest is stably maintained.

The transformation may be performed using a suitable vector introduction technique selected depending on the host cell so that the gene of the interest or a recombinant vector containing the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or any combination thereof, without being limited thereto. As long as the transformed gene may be expressed within the host cell, it may be included without limitation, regardless of whether or not the gene is inserted into the chromosome of the host cell or located outside the chromosome.

The term "transformant" includes cells transfected, transformed, or infected with the recombinant vector of the present invention *in vivo* or *in vitro,* and may be used interchangeably with the term "recombinant host cell", "recombinant cell", or "recombinant microorganism".

According to one embodiment of the present invention, the transformant is a 1,4-butanediol-producing microorganism, and is not particularly limited as long as it is a microorganism known in the art.

More specifically, the 1,4-butanediol-producing microorganism may be an *Escherichia* sp. or *Corynebacterium* sp. strain.

Examples of the *Escherichia* sp. strain include, but are not limited to, *Escherichia coli, Escherichia albertii, Escherichia blattae, Escherichia fergusonii, Escherichia hermannii, Escherichia vulneris,* and the like.

Examples of the *Corynebacterium* sp. strain include, but are not limited to, *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacterium pseudopelargi, Corynebacterium flavescens,* and the like.

Another aspect of the present invention provides a method for producing 1,4-butanediol, comprising steps of: culturing the above-described microorganism in a medium; and recovering 1,4-butanediol from the microorganism or the medium in which the microorganism has been cultured.

The culturing may be performed using a suitable medium and culture conditions known in the art, and any person skilled in the art may easily adjust and use the medium and the culture conditions. Specifically, the medium may be a liquid medium, without being limited thereto. The culturing method include may include, for example, batch culture, continuous culture, fed-batch culture, or any combination thereof, without being limited thereto.

According to one embodiment of the present invention, the medium should meet the requirements of a specific strain in a proper manner, and may be appropriately modified by a person skilled in the art. For the culture media for an *Escherichia* sp. strain and a *Corynebacterium* sp. strain, reference may be made to a known document (Manual of Methods for General Bacteriology, American Society for Bacteriology, Washington D.C., USA, 1981), without being limited thereto.

According to one embodiment of the present invention, the medium may contain various carbon sources, nitrogen sources, and trace element components. Examples of the carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of the nitrogen sources that may be used include peptone, yeast extract, meat extract, malt extract, corn steep liquor, soybean meal, and urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be added to the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering 1,4-butanediol from the cultured mutant strain or the medium in which the mutant strain has been cultured, the produced 1,4-butanediol may be collected or recovered from the medium using a suitable method known in the art depending on the culture method. Examples of the method include, but are not limited to, centrifugation, filtration, extraction, spraying, drying, evaporation, precipitation, crystallization, electrophoresis, fractional dissolution (e.g., ammonium sulfate precipitation), and chromatography (e.g., ion exchange, affinity, hydrophobicity and size exclusion).

According to one embodiment of the present disclosure, the step of recovering 1,4-butanediol may be performed by centrifuging the culture medium at a low speed to remove biomass and separating the obtained supernatant through ionexchange chromatography.

According to one embodiment of the present disclosure, the step of recovering 1,4-butanediol may include a process of purifying 1,4-butanediol.

### Advantageous Effects

The microorganism according to the present invention is able to efficiently produce 1,4-butanediol using ornithine as a carbon source, unlike naturally occurring microorganisms.

### Brief Description of Drawings

FIG. 1 shows the results of evaluating the conversion of ornithine to 4-aminobutyric acid (GABA) in three *E. coli* strains expressing davB and davA according to one example of the present invention.
FIG. 2 shows the results of evaluating the conversion of ornithine to 4-aminobutyric acid (GABA) in three *E. coli* strains expressing LAAO/MOG according to one example of the present invention.

### Mode for Invention

Hereinafter, the present invention will be described in more detail. However, this description is provided by way of example only to aid the understanding of the present invention, and the scope of the present invention is not limited by this illustrative description.

### Example 1. Evaluation of Activity of Substrate in E. coli Expressing davB and davA Genes

### 1-1. Construction of Vector Expressing davB and davA

davB and davA were amplified by PCR using the chromosomal DNA of a *Pseudomonas putida* strain as a template and primers, and then cloned into the plasmid pKM212-MCS. Here, a 6x His-tag was inserted into the N-terminus of the davB gene. The resulting vector was named pKM212-DavBHisA.

Here, the Wizard Genomic DNA Purification Kit (Promega, USA) was used to extract DNA from each strain. The nucleotide sequences of the amplified genes davB and davA are shown in Table 1 below, and information on the primers and plasmids used is shown in Tables 2 and 3 below, respectively.

Using a thermocycler (TP600, TAKARA BIO Inc., Japan) and a reaction solution containing 100 pM of each deoxynucleotide triphosphate (dATP, dCTP, dGTP, dTTP), 1 pM of oligonucleotide, and 10 ng of template DNA, PCR was performed in the presence of one unit of a PrimeSTAR Max DNA polymerase (Takara, Japan) for 25 to 30 cycles, each consisting of 30 sec at 94°C, 30 sec at 55°C, and 1 min at 72°C.

The pKM212-DavBHisA vector was transformed into *E*. *coli* DH5a (HIT competent cells^{™}, Cat No. RH618), which was then streaked on an LB-agar plate containing 50 ug/ml of kanamycin, and cultured at 37°C for 24 hours. After the finally formed colonies were isolated and it was checked whether the inserts would be exactly present in the vector, the vector was isolated and used to produce *E*. *coli* expressing davB and davA.

**[Table 1]**

| SEQ ID NO. | Gene | Nucleotide sequence (5'→3') |
|---|---|---|
| 1 | davB | atgaacaagaagaaccgccaccccgccgacggcaagaagccgatcaccattttc ggcccggacttcccttttgctttcgacgactggctggaacacccggcaggcctg ggcagcattccggctgagcgccatggggaagaggtggccattgtcggtgccggt atcgccggcctggtagcggcctacgagctgatgaagctgggcctcaagccggtg |
| | | gtgtacgaggcttccaagctgggcggccggctgcgctcgcaagccttcaatggc actgacgggatcgttgccgagctgggtggcatgcgcttcccggtgtcgtccacc gccttctaccactacgtcgacaagctgggcctggagaccaagcccttccccaac ccgctgaccccggcttcgggcagcacggtgatcgacctggaaggccagacctac tacgccgagaagcccaccgacctgccgcaactgtttcatgaggtagccgacgct tgggccgatgctctggagagcggtgcgcagttcgccgatatccagcaggccatc cgcgaccgtgatgtaccgcgcctgaaggaactctggaacaagctggtgccactg tgggacgaccgcaccttctacgacttcgtcgccacctcgcgctcttttgccaag ctgagcttccagcaccgcgaagtgttcggccaggtcggtttcggcaccggcggt tgggactcggacttccccaactcgatgctggaaatcttccgcgtggtgatgacc aactgcgacgaccaccagcacctggtggtcgggggcgtggaacaagtgccacaa ggcatctggcgcgacgtaccggaacgctgcgtgcattggccagagggcaccagc ctgagcacgctgcatggcggcgcaccgcgtaccggggtcaagcgcattgcccgc gccgccgatggccgcctggcggtcaccgacaactggggcgatacccgccactac agcgcagtactcgccacctgccagacctggttgctgaccacccagatcgactgc gaggaatcgctgttctcgcaaaagatgtggatggccctggaccgtacccgctac atgcagtcgtcgaaaaccttcgtcatggtcgaccgcccgttctggaaggacaag gacccggaaaccggccgtgacctgctgagcatgaccctcaccgaccgcctcacc cgcggcacttacctgttcgacaacggcaacgacaagcccggggtgatctgcctg tcgtactcgtggatgagcgacgcgctgaagatgctgccgcacccggtggaaaag cgcgtacaactggccctggatgcgctgaagaagatctacccgaagaccgatatc gccgggcacatcatcggcgacccgatcacggtttcctgggaggccgacccgtac ttcctcggcgccttcaaaggcgcgcttccgggccattaccgctacaaccagcgc atgtacgcgcacttcatgcagcaggacatgccggcggagcagcgcggtatcttc attgccggtgacgacgtgtcatggacccccgcctgggttgaaggcgcggtgcag |
| | | acgtcgctgaatgcggtgtggggtatcatgaaccactttggtggccacacccac cccgacaaccccggcccgggcgatgtgttcaacgaaatcggcccgatcgccctg gcggattga |
| 3 | davA | atgcgcatcgctctgtaccagggcgcacccaagccactggatgtgcccggcaac ctgcaacggctgcgccaccaggcgcagttggcagccgaccgcggcgcacagttg ctggtgtgcccggagatgttcctgtccggctacaacatcggcctggcccaggtc gagcgcctggccgaggccgccgatggcccggcagccatgacggtggtggagatt gcccaggcgcaccgtatcgccattgtctatggctacccggagcgcggcgatgac ggggcgatctacaacagcgtgcagctgatcgatgcgcatggccgcagcctgagc aattaccgcaagacccacctgttcggtgaactggaccgctcgatgttcagccct ggtgcggaccacttcccggtggtggaactggaaggctggaaggttggcctgctg atctgctacgacatcgagttcccggagaacgcccgacgcctggcgctggacggc gccgagctgatcctggtgccgacggcgaacatgacgccgtacgactttacctgc caggtgaccgtgagggcacgggcgcaggaaaaccagtgctacctggtatatgcc aactactgcggcgcggaagacgagatcgagtattgcgggcagagcagcatcatc ggcccggatggcagcttgctggccatggccgggcgggatgagtgccagttgttg gcagagctcgagcatgagcgggtggtgcaggggcgcagggcgtttccctacctg accgatttgcgccaggagctgcacctgcgtaaaggctga |

**[Table 2]**

| SEQ ID NO. | Primer name | Primer sequence (5'→3') |
|---|---|---|
| 13 | DavBHis-F-BamHI | ggatcctttcacacaggaaacagaccatgcaccatcatcaccatc acatgaacaagaagaaccgccac |
| 14 | DavA-R-HindIII | aagctttcagcctttacgcaggtgc |

**[Table 3]**

| Plasmid name | Description |
|---|---|
| pKM212-MCS | pBBR1MCS2 derivative; tac promoter, *Ralstonia eutropha* PHA biosynthesis genes transcription terminator; Km^{R} (Park et al., Metab Eng. 2013 20-28) |
| pKM212-DavBHisA | pKM212-MCS derivative; *Pseudomonas putida* DavB (N-terminal 6xHis-tag) and DavA; Km^{R} |

### 1-2. Construction of E. coli Expressing davB and davA

The pKM212-DavBHisA vector was introduced into competent cells of each of *E. coli* XL1-Blue (HIT Competent cells^{™}, Cat No. RH118), *E. coli* WL3110 (Park et al., Proc Natl Acad Sci USA. 2007 104(19):7797-802), and *E. coli* DH5a (HIT Competent cells^{™}, Cat No. RH618), by heat shock at 42°C for 1 minute and 30 seconds. Then, each *E. coli* strain was streaked on an LB agar plate containing 50 ug/ml of kanamycin, and cultured at 37°C for 24 hours. The selected colonies were finally analyzed by PCR and sequencing to determine whether the davB and davA genes were introduced as intended. In addition, they were named *E. coli* XL1-Blue harboring pKM212DavBHisA, *E. coli* WL3110 harboring pKM212DavBHisA, and *E. coli* DH5a harboring pKM212DavBHisA, respectively.

### 1-3. Evaluation of Activity of E. coli Expressing davB and davA for Ornithine

The present inventors evaluated the conversion of ornithine to 4-aminobutyric acid (GABA) in each of *E. coli* XL1-Blue harboring pKM212DavBHisA, *E. coli* WL3110 harboring pKM212DavBHisA, and *E. coli* DH5a harboring pKM212DavBHisA, into which the davB and davA gene have been introduced.

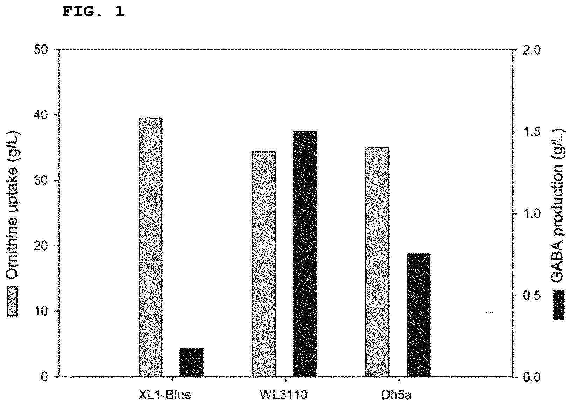

Each strain was inoculated into a 50 mL flask containing 5 mL of MR medium (containing 200 g/L ornithine) and then cultured at 37°C for 96 hours. After completion of the culturing, the culture was centrifuged to separate the supernatant and the cell pellet, and the supernatant was treated with DEEMM (Kim et al., J. Mol. Catal. B: Enzym. 2015 115:151-154), and filtered through a 0.22 um filter. Then, the ornithine and GABA contents in the supernatant were analyzed using high-performance liquid chromatography (HPLC) (Agilent, 1260 infinity II) equipped with a column (C18 column, RStech) and a variable wavelength detector (VWD). The results are shown in FIG. 1.

As shown in FIG. 1, it was confirmed that GABA production varied depending on the type of host strain, and in particular, *E. coli* WL3110 expressing davB and davA had the highest bioconversion rate of ornithine.

### Example 2. Evaluation of Activity of Substrate in E. coli Expressing LAAO/MOG Genes

L-amino acid oxidase/monooxygenase (LAAO/MOG) derived from *Pseudomonas* is known to exhibit not only oxidase activity but also lysine 2-monooxygenase activity that converts ornithine to 4-aminobutyric acid (GABA). It was reported that, when cysteine at position 254 in the amino acid sequence of LAAO/MOG was substituted with isoleucine or leucine, the activity of the substrate for ornithine increased (FEES Open Bio, 2014 4:220-228).

Accordingly, *E. coli* expressing LAAO/MOG was constructed to produce GABA from ornithine, and the activity of the substrate for ornithine therein was evaluated.

### 2-1. Construction of Vectors Expressing LAAO/MOG

LAAO/MOG from the *Pseudomonas* sp. AIU 813 strain was synthesized (Bioneer) through codon optimization based on *E*. *coli* and cloned into plasmid pKM212-MCS using primers. The synthesized product was named pKM212-LAAO/MOG.

To induce mutation in the synthesized LAAO/MOG gene, LAAO/MOG(C254I) and LAAO/MOG(C254L) genes were synthesized by substituting the amino acid cysteine at position 254 with each of isoleucine and leucine, and then each cloned into the plasmid pKM212-MCS using primers. The resulting vectors were named pKM212-LAAO/MOG(C254I) and pKM212-LAAO/MOG(C254L), respectively.

Here, the Wizard Genomic DNA Purification Kit (Promega, USA) was used to extract DNA from each strain. The nucleotide sequence of the amplified gene LAAO/MOG is shown in Table 4 below, and information on the primers and plasmids used is shown in Tables 5 and 6 below, respectively.

Using a thermocycler (TP600, TAKARA BIO Inc., Japan) and a reaction solution containing 100 pM of each deoxynucleotide triphosphate (dATP, dCTP, dGTP, dTTP), 1 pM of oligonucleotide, and 10 ng of template DNA, PCR was performed in the presence of one unit of a PrimeSTAR Max DNA polymerase (Takara, Japan) for 25 to 30 cycles, each consisting of 30 sec at 94°C, 30 sec at 55°C, and 1 min at 72°C.

Each of the pKM212-LAAO/MOG, pKM212-LAAO/MOG(C254I) and pKM212-LAAO/MOG(C254L) vectors was transformed into *E*. *coli* WL3110 (Park et al., Proc Natl Acad Sci USA. 2007 104(19):7797-802), which was then streaked on an LB-agar plate containing 50 ug/ml of kanamycin, and cultured at 37°C for 24 hours. After the finally formed colonies were isolated and it was checked whether the insert would be exactly present in the vectors, each of the vectors was isolated and used to produce *E. coli* expressing LAAO/MOG.

**[Table 4]**

| SEQ ID NO. | Gene | Nucleotide sequence (5'→3') |
|---|---|---|
| 15 | LAAO/MOG | atgaacaagaataaccgccatcctgcagatgggaaaaaaccgatcacaattt ttggcccggacttcccttttgcctttgatgattggctggaacacccagctgg |
| | | actgggtagcattccagcagcgcgacatggtgaggaagttgctatcgtgggt gccggcattgccgggctggtggcggcctacgaactgatgaagctgggcttaa aaccggtggtctatgaagcgtcgaaaatgggtgggcggctgagatcacaggc attcaatggcacggatggaataatagccgagctgggaggaatgcgctttccg gtgtcttccacggcgttctatcactacgttgataagttaggcctagaaacga agccttttcctaaccccctcactcctgcctcacgttcaacggtaatagatct ggaagggcagacctattacgcggaaaaagcagccgatttgccagcccttttt caggaagttaccgatgcctgggctgacgccctggaaagcggtgcgcgctttg gtgatatccagcaagcaattagggatcgtgatgtgcctcgcttgaaagaact gtggaacaccctggttccactgtgggacgaccgtactttttatgattttgtc gccacatccaaagcttttgccaaactgtcttttcaacaccgcgaggttttcg gccaggttggctttggcaccggtggttgggattcggatttccccaattcgat gctggaaatcttccgtgtagttatgaccaattgcgacgaccatcaacatctg gtggttggcggcgtcgagcaagtaccgcagggtatctggaggcatgtgccag agcgttgtgcgcattggcccgaaggtacatcactgagttctctgcacggcgg tgctccgagaaccggcgtgaaacgcattgcccgggcaagtgacggccgactg gcagtcacagacaactggggcgactgcagacattacgcagccgtattaacga catgtcagagttggctgctgaccactcagatcgactgtgaagaaagcttgtt ttcacagaagatgtggatggctctagatagaactcgttatatgcaatcttcg aaaacctttgtaatggtcgaccggccgttttggaaagacaaagacccagaaa ctggtcgcgacctgatgagcatgacgcttactgaccgcctgacccgtggcac ctatttattcgataatggcgacgacaaaccaggagtaatctgcttaagttat gcatggatgagcgatgctctgaaaatgctcccccatccggttgaaaaacgtg tccaactggcattagatgcattgaaaaagatctacccgaaaacggatattgc cgggcatattattggcgaccctattacaatttcttgggaagctgatcctcac |
| | | ttcctcggtgcattcaaaggcgcgctgccgggccactatcgctataaccagc ggatgtatgcgcattttatgcaggcacagatgccagtagagcagcgtggaat tttcattgctggcgacgacgtgtcgtggaccccggcatgggtcgaaggcgcg gtgcaaacatcgctcaatgcagtgtggggtataatgaatcattttggtggca aaacccatgctgacaatccgggccctggagatgtgttcgatgagatcggcca aatagcgctggcggactaa |
| 16 | LAAO/MOG (C254I) | atgaacaagaataaccgccatcctgcagatgggaaaaaaccgatcacaattt ttggcccggacttcccttttgcctttgatgattggctggaacacccagctgg actgggtagcattccagcagcgcgacatggtgaggaagttgctatcgtgggt gccggcattgccgggctggtggcggcctacgaactgatgaagctgggcttaa aaccggtggtctatgaagcgtcgaaaatgggtgggcggctgagatcacaggc attcaatggcacggatggaataatagccgagctgggaggaatgcgctttccg gtgtcttccacggcgttctatcactacgttgataagttaggcctagaaacga agccttttcctaaccccctcactcctgcctcacgttcaacggtaatagatct ggaagggcagacctattacgcggaaaaagcagccgatttgccagcccttttt caggaagttaccgatgcctgggctgacgccctggaaagcggtgcgcgctttg gtgatatccagcaagcaattagggatcgtgatgtgcctcgcttgaaagaact gtggaacaccctggttccactgtgggacgaccgtactttttatgattttgtc gccacatccaaagcttttgccaaactgtcttttcaacaccgcgaggttttcg gccaggttggctttggcaccggtggttgggattcggatttccccaattcgat gctggaaatcttccgtgtagttatgaccaatattgacgaccatcaacatctg gtggttggcggcgtcgagcaagtaccgcagggtatctggaggcatgtgccag agcgttgtgcgcattggcccgaaggtacatcactgagttctctgcacggcgg tgctccgagaaccggcgtgaaacgcattgcccgggcaagtgacggccgactg gcagtcacagacaactggggcgactgcagacattacgcagccgtattaacga |
| | | catgtcagagttggctgctgaccactcagatcgactgtgaagaaagcttgtt ttcacagaagatgtggatggctctagatagaactcgttatatgcaatcttcg aaaacctttgtaatggtcgaccggccgttttggaaagacaaagacccagaaa ctggtcgcgacctgatgagcatgacgcttactgaccgcctgacccgtggcac ctatttattcgataatggcgacgacaaaccaggagtaatctgcttaagttat gcatggatgagcgatgctctgaaaatgctcccccatccggttgaaaaacgtg tccaactggcattagatgcattgaaaaagatctacccgaaaacggatattgc cgggcatattattggcgaccctattacaatttcttgggaagctgatcctcac ttcctcggtgcattcaaaggcgcgctgccgggccactatcgctataaccagc ggatgtatgcgcattttatgcaggcacagatgccagtagagcagcgtggaat tttcattgctggcgacgacgtgtcgtggaccccggcatgggtcgaaggcgcg gtgcaaacatcgctcaatgcagtgtggggtataatgaatcattttggtggca aaacccatgctgacaatccgggccctggagatgtgttcgatgagatcggcca aatagcgctggcggactaa |
| 17 | LAAO/MOG (C254L) | atgaacaagaataaccgccatcctgcagatgggaaaaaaccgatcacaattt ttggcccggacttcccttttgcctttgatgattggctggaacacccagctgg actgggtagcattccagcagcgcgacatggtgaggaagttgctatcgtgggt gccggcattgccgggctggtggcggcctacgaactgatgaagctgggcttaa aaccggtggtctatgaagcgtcgaaaatgggtgggcggctgagatcacaggc attcaatggcacggatggaataatagccgagctgggaggaatgcgctttccg gtgtcttccacggcgttctatcactacgttgataagttaggcctagaaacga agccttttcctaaccccctcactcctgcctcacgttcaacggtaatagatct ggaagggcagacctattacgcggaaaaagcagccgatttgccagcccttttt caggaagttaccgatgcctgggctgacgccctggaaagcggtgcgcgctttg gtgatatccagcaagcaattagggatcgtgatgtgcctcgcttgaaagaact |
| | | gtggaacaccctggttccactgtgggacgaccgtactttttatgattttgtc gccacatccaaagcttttgccaaactgtcttttcaacaccgcgaggttttcg gccaggttggctttggcaccggtggttgggattcggatttccccaattcgat gctggaaatcttccgtgtagttatgaccaatcttgacgaccatcaacatctg gtggttggcggcgtcgagcaagtaccgcagggtatctggaggcatgtgccag agcgttgtgcgcattggcccgaaggtacatcactgagttctctgcacggcgg tgctccgagaaccggcgtgaaacgcattgcccgggcaagtgacggccgactg gcagtcacagacaactggggcgactgcagacattacgcagccgtattaacga catgtcagagttggctgctgaccactcagatcgactgtgaagaaagcttgtt ttcacagaagatgtggatggctctagatagaactcgttatatgcaatcttcg aaaacctttgtaatggtcgaccggccgttttggaaagacaaagacccagaaa ctggtcgcgacctgatgagcatgacgcttactgaccgcctgacccgtggcac ctatttattcgataatggcgacgacaaaccaggagtaatctgcttaagttat gcatggatgagcgatgctctgaaaatgctcccccatccggttgaaaaacgtg tccaactggcattagatgcattgaaaaagatctacccgaaaacggatattgc cgggcatattattggcgaccctattacaatttcttgggaagctgatcctcac ttcctcggtgcattcaaaggcgcgctgccgggccactatcgctataaccagc ggatgtatgcgcattttatgcaggcacagatgccagtagagcagcgtggaat tttcattgctggcgacgacgtgtcgtggaccccggcatgggtcgaaggcgcg gtgcaaacatcgctcaatgcagtgtggggtataatgaatcattttggtggca aaacccatgctgacaatccgggccctggagatgtgttcgatgagatcggcca aatagcgctggcggactaa |

**[Table 5]**

| SEQ ID NO. | Primer name | Nucleotide sequence (5'→3') |
|---|---|---|
| 18 | LAAO/MOG-EroRI-F | gaattcatgaacaagaataaccgcc |
| 19 | LAAO/MOG-KpnI-R | ggtaccttagtccgccagcgctatttg |

**[Table 6]**

| Plasmid name | Description |
|---|---|
| pKM212-MCS | pBBR1MCS2 derivative; tac promoter, *Ralstonia eutropha* PHA biosynthesis genes transcription terminator; Km^{R} (Park et al., Metab Eng. 2013 20-28) |
| pKM212-LAAO/MOG | pKM212-MCS derivative; *Pseudomonas* sp. AIU 813 1-amino acid oxidase/monooxygenase (*E. coli* codon-optimized); Km^{R} |
| pKM212-LAAO/MOG(C254I) | pKM212-MCS derivative; *Pseudomonas* sp. AIU 813 1-amino acid oxidase/monooxygenase variant (C254I; *E. coli* i codon-optimized); Km^{R} |
| pKM212-LAAO/MOG(C254L) | pKM212-MCS derivative; *Pseudomonas* sp. AIU 813 1-amino acid oxidase/monooxygenase variant (C254L; *E. coli* codon-optimized); Km^{R} |

### 2-2. Construction of E. coli Expressing LAAO/MOG

Each of the pKM212-LAAO/MOG, pKM212-LAAO/MOG(C254I) and pKM212-LAAO/MOG(C254L) vectors was introduced into competent cells of *E. coli* WL3110 by heat shock at 42°C for 1 minute and 30 seconds. Then, each *E. coli* strain was streaked on an LB agar plate containing 50 ug/ml of kanamycin, and cultured at 37°C for 24 hours. The selected colonies were finally analyzed by PCR and sequencing to determine whether the LAAO/MOG, LAAO/MOG(C254I) or LAAO/MOG(C254L) gene was introduced as intended. *E. coli* strains into which the LAAO/MOG gene has been introduced were finally constructed and these were named *E. coli* WL3110 harboring pKM212LAAO/MOG, *E. coli* WL3110 harboring pKM212LAAO/MOG(C254I), and *E. coli* WL3110 harboring pKM212LAAO/MOG(C254L).

### 2-3. Evaluation of Activity of E. coli Strains Expressing LAAO/MOG for Ornithine

The present inventors evaluated the conversion of ornithine to 4-aminobutyric acid (GABA) in each of *E. coli* WL3110 harboring pKM212LAAO/MOG, *E. coli* WL3110 harboring pKM212LAAO/MOG(C254I), and *E. coli* WL3110 harboring pKM212LAAO/MOG(C254L), into which the LAAO/MOG gene has been introduced.

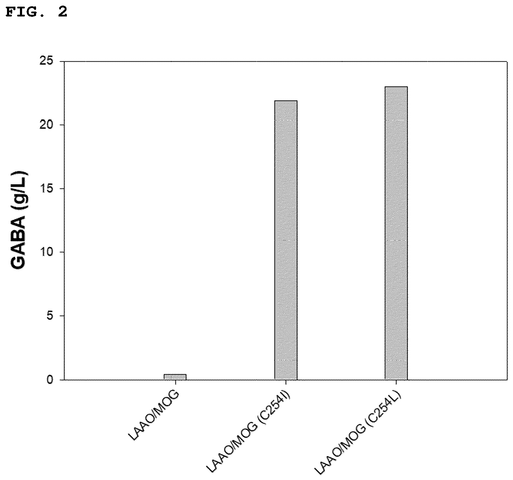

Each strain was inoculated into a 1.5 mL e-tube containing 1 mL of medium (containing Tris-HCl (pH 7.6) and 500 mM ornithine) and cultured at 37°C for 96 hours. After completion of the culturing, the culture was centrifuged to separate the supernatant and the cell pellet, and the supernatant was treated with DEEMM (Kim et al., J. Mol. Catal. B: Enzym. 2015 115:151-154), and filtered through a 0.22 um filter. Then, the ornithine and GABA contents in the supernatant were analyzed using high-performance liquid chromatography (HPLC) (Agilent, 1260 infinity II) equipped with a column (C18 column, RStech) and a variable wavelength detector (VWD). The results are shown in FIG. 2.

As shown in Fig. 2, it was confirmed that when the mutated LAAO/MOG was expressed, the bioconversion rate of ornithine was higher than when the wild-type LAAO/MOG was expressed, indicating that GABA was produced in a higher yield.

### Example 3. Evaluation of 1,4-Butanediol Productivity in Corynebacterium glutamicum with Ornithine-1,4-Butanediol Pathway Introduced

### 3-1. Construction of Vector Expressing davB, davA, gabT, yahK and car

Using the chromosomal DNA of the *Corynebacterium glutamicum* ATCC13032 strain as a template, PCR amplification was performed using primers 1 and 2, primers 3 and 4, primers 7 and 8, and primers 11 and 12. Using the vector pKM212-DavBHisA expressing davB and davA constructed in Example 1 as a template, PCR amplification was performed using primers 5 and 6 and primers 9 and 10. The resulting PCR products were amplified by crossover PCR and then inserted into the restriction enzymes HindIII (NEB, R3104S) and XbaI (NEB, R0145L) sites of the pK19mobSacB vector (Jaeger et al., Journal of Bacteriology. 1992 174: 5462-65). The resulting vector was named pK19ms/davBA.

Using the chromosomal DNA of the *Corynebacterium glutamicum* ATCC13032 strain as a template, PCR amplification was performed using primers 13 and 14, primers 15 and 16, primers 19 and 20, primers 21 and 22, and primers 23 and 24. Using the chromosomal DNA of the *Escherichia coli* K-12 MG1655 strain containing GabT (derived from *Corynebacterium glutamicum*) and YahK (derived from *Escherichia coli*) as a template, PCR amplification was performed using primers 17 and 18. The resulting PCR products were amplified by crossover PCR and then inserted into the restriction enzyme HindIII and XbaI sites of the pK19mobSacB vector. The resulting vector was named pK19ms/yahK-gabT.

To insert the sfp gene required for activating the enzymatic activity of the car gene, using the chromosomal DNA of the *Corynebacterium glutamicum* ATCC13032 strain as a template, PCR amplification was performed using primers 25 and 26, primers 27 and 28, and primers 31 and 32. Using the *E. coli* expression vector pKE112CAR3pptase (Polymers (Basel). 2019 11(7):1184) containing sfp (derived from *Bacillus subtilis*) as a template, PCR amplification was performed using primers 29 and 30. The resulting PCR products were amplified by crossover PCR and then inserted into the restriction enzymes HindIII and XbaI sites of the pK19mobSacB vector. The resulting vector was named pK19ms/sfp.

Using the chromosomal DNA of the *Corynebacterium glutamicum* ATCC13032 strain as a template, PCR amplification was performed using primers 33 and 34, primers 35 and 36, and primers 39 and 40. Using the *E. coli* expression vector pKE112CAR3pptase (Polymers (Basel). 2019 11(7):1184) containing car (derived from *Mycobacterium abscessus*) as a template, PCR amplification was performed using primers 37 and 38. The resulting PCR products were amplified by crossover PCR and then inserted into the restriction enzymes HindIII and XbaI sites of the pK19mobSacB vector. The resulting vector was named pK19ms/car.

Here, the Wizard Genomic DNA Purification Kit (Promega, USA) was used to extract DNA from each strain. The nucleotide sequences of the amplified genes gabT, yahK, and car are shown in Table 7 below, and the primers in Table 8 below were used for PCR.

Using a thermocycler (TP600, TAKARA BIO Inc., Japan) and a reaction solution containing 100 pM of each deoxynucleotide triphosphate (dATP, dCTP, dGTP, dTTP), 1 pM of oligonucleotide, and 10 ng of template DNA, PCR was performed in the presence of one unit of a PrimeSTAR Max DNA polymerase (Takara, Japan) for 25 to 30 cycles, each consisting of 30 sec at 94°C, 30 sec at 58°C, and 1 min at 72°C.

Each of the pK19ms/davBA, pK19ms/yahK-gabT, pK19ms/sfp and pK19ms/car vectors was transformed into *E*. *coli* DH5a (HIT Competent cells^{™}, Cat No. RH618), which was then streaked on an LB-agar plate containing 50 ug/ml of kanamycin, and cultured at 37°C for 24 hours. After the finally formed colonies were isolated and it was checked whether the inserts would be exactly present in the vector, the vector was isolated and used to produce *Corynebacterium glutamicum* with the ornithine-1,4-butanediol pathway introduced.

**[Table 7]**

| SEQ ID NO. | Gene | Nucleotide sequence (5'-3') |
|---|---|---|
| 5 | gabT | gtggaagatctctcataccgcatcccgcagtcgcgcaccgtggccgagcaggtg ccagggccgaagtcgaaagcgctggatgagcgtcgacaagcagcagtagcacga gcacttgcaccgggtctgcctggatacgtggtggacgcagacggtggcatcttg gctgacgcggacggcaaccgtttcatcgacctggcctccggcatcgccgtgacc acggtcggcggatccaacgcggccgtcgcgaaagccgtcggcgccgcagctgcc cgcttcacccacacctgcttcatggtctcaccttatgaaacttacgtggccatg gcggagagactcaacgccttgactccaggcgatcacgacaagaagagcgcgctg tttaactctggcgccgaagccgtggaaaacgccgtcaaggtggcacgcgcctac accggcaagggcgcggtcgtggtgttcgacaacgcgtaccacggacggaccaac ctcaccatggcgatgaccgcgaagaaccgcccatacaagtccggattcggacca ctagccgcagacgtctaccgtgcaccaatgtcttacccactgcgcgacggactg |
| | | tccggcccggaagccgcagagcgcgcgatctccgtgatcgaatcccaggtcgga gccgaaaacctcgcctgcgtggtcattgaaccgatccagggcgaaggcggattc atcgtccccgcaccaggattcctcgcagccatttccacctggtgccgcgagaac gacgtggtgttcatcgccgatgaaatccaatctggcttcctgcgcaccggcgac tggttcgccagcgacgcagaaggtgtgatccccgacgtcatcaccaccgcaaaa ggcatcgccggcggcatgccactatccgcagtgaccggccgcgcagaaatcatg gacgcacccggccccggcgcgctcggcggaacctacggcggaaaccccgttgct tgcgccgcggcacttgcagccattgaagtgatggaacaagccgaccttaagacc cgcgcgcaagaaatcgagaccatcatccgcgatgaattcgcgcagctgagtgcc ttcccggaggtcgccgaaatccgcggccgcggagcaatgatggccattgagctt atcgacgctaccggccgcccgaacgcagctttaaccgccgcagtggctgcgcgc gcaaaagctgaaggtgtgctgctgctgacttgcggcaccgatggcaacgtcatc cgcctgctgccaccactggtcattgcagaggacactctccgtgatggtcttcag gtgttagtcgcagccctagagcgcgaaaccgcgcaccagaaggtgggctaa |
| 7 | yahK | atgaagatcaaagctgttggtgcatattccgctaaacaaccacttgaaccgatg gatatcacccggcgtgaaccgggaccgaatgatgtcaaaatcgaaatcgcttac tgtggcgtttgccattccgatctccaccaggtccgttccgagtgggcggggacg gtttacccctgcgtgccgggtcatgaaattgtggggcgtgtggtagccgttggt gatcaggtagaaaaatatgcgccgggcgatctggtcggtgtcggctgcattgtc gacagttgtaaacattgcgaagagtgtgaagacgggttggaaaactactgtgat cacatgaccggcacctataactcgccgacgccggacgaaccgggccatactctg ggcggctactcacaacagatcgtcgttcatgagcgatatgttctgcgtattcgt cacccgcaagagcagctggcggcggtggctcctttgttgtgtgcagggatcacc acgtattcgccgctacgtcactggcaggccgggccgggtaaaaaagtgggcgtg gtcggcatcggcggtctgggacatatggggattaagctggcccacgcgatgggg |
| | | gcacatgtggtggcatttaccacttctgaggcaaaacgcgaagcggcaaaagcc ctgggggccgatgaagttgttaactcacgcaatgccgatgagatggcggctcat ctgaagagtttcgatttcattttgaatacagtagctgcgccacataatctcgac gattttaccaccttgctgaagcgtgatggcaccatgacgctggttggtgcgcct gcgacaccgcataaatcgccggaagttttcaacctgatcatgaaacgccgtgcg atagccggttctatgattggcggcattccagaaactcaggagatgctcgatttt tgcgccgaacatggcatcgtggctgatatagagatgattcgggccgatcaaatt aatgaagcctatgagcgaatgctgcgcggtgatgtgaaatatcgttttgttatc gataatcgcacactaacagactga |
| 9 | car | atgactgaaacgatctccacagcggctgtccccactacggatctcgaagagcag gtgaagcgacgcatcgagcaggtcgtgtccaacgatccgcagctggcggcgctt ctcccggaagattcggtcaccgaggcggtcaacgagcccgatctaccgctggtc gaggtgatcaggcgactgctggagggctacggtgaccgcccggcactcggccag cgcgccttcgagttcgtcaccggggacgacggtgcgaccgtgatcgcgctgaag cccgaatacaccaccgtctcctaccgcgagttgtgggaacgtgccgaggctatc gctgccgcgtggcacgagcagggcatccgtgacggcgacttcgtcgctcagttg ggtttcaccagcacggacttcgcgtcgctcgacgtcgcgggattgcgtctgggc accgtctcggtgcccctgcagacgggcgcgtcgctgcagcagcgcaacgcgatt ctcgaagagacccggcccgcagtctttgccgcgagtatcgaataccttgatgcc gccgtcgattcggtgcttgcgaccccctcggtgcgactcctctcggttttcgac tatcacgcggaggtcgacagccagcgcgaagcgctggaggctgtgcgggcccgg cttgagagtgccggccggacgatcgtcgtcgaggccctggcggaggctctcgcg cgggggcgggacctgcccgccgcgccgctgcccagtgcagatcccgatgccttg cgtctgctcatctacacctccggcagcaccggtacccccaagggcgccatgtat ccgcaatggctggtcgccaacttgtggcagaagaagtggctcaccgacgatgtg |
| | | attccgtccataggcgtgaacttcatgcccatgagccacctggcgggtcgcctc actctcatgggcaccctttccggtggcggaaccgcctactacatcgcttcgagc gatctttcgactttcttcgaggacatcgcgctcatccgcccctccgaagtgctc ttcgtgccgcgtgtggtggagatggtgttccagcgttttcaggcagaattggac cggtcccttgccccgggtgagagcaactccgagatcgcggagcgaatcaaggtc cgcatccgggaacaggacttcggcgggcgtgtgctcagtgctggctccgggtcg gccccgttgtctcctgagatgacggagttcatggagtcgctgctgcaggtgccg ttgcgcgacgggtatgggtccaccgaggccggtggtgtgtggcgtgacggagtc ctgcagcgtccgcccgtcaccgactacaagctggttgacgttccggaactcgga tacttcaccacagattcgccgcatccccgtggcgagctgcggttgaagtcggag acgatgttccccggctactacaagcgcccggagaccactgccgatgtcttcgat gacgaggggtactacaagaccggtgacgtggtcgccgagctcgggccggatcac ctcaagtacctcgaccgcgtcaagaacgtcctcaagctcgcgcagggagagttt gtcgcggtgtcaaagctggaggccgcttacaccggcagcccgctggtccggcag atctttgtgtacgggaacagtgaacgctcgttcctgctggctgtcgtggtcccg acacccgaagtccttgagcggtacgcagattcgccagatgcgctcaagcccttg atccaggattcgctgcagcaggtcgccaaggacgcggagctgcaatcctatgag ataccgcgcgacttcatcgttgagacggtgccgttcaccgtcgagtccggattg ctatcggacgcgcgaaagctgctgcgccccaagctgaaggatcactacggagag aggctggaggcgctgtacgccgaactggcggaaagccagaatgagcggctgcgc cagttggccagggaggcagccacgcgcccggtcctggagacggtgaccgatgcg gccgccgcgctgctgggcgcatcgtcctcggatctggctcctgatgtgcgattc atcgacctcggtggcgactcactgtcggcgctgtcgtactccgagctgctgcgc gacatctttgaggtggacgttccggtgggcgtcatcaacagcgtcgccaacgac cttgccgcgatcgcccggcacatcgaggcgcagcggaccggcgccgctacgcag |
| | | ccgacctttgcgtcggtccacggcaaggacgcgacggtcatcaccgccggtgaa ctcaccctcgacaagttcttggacgagtcactgttgaaagcggccaaggacgtt cagccggcaacggccgatgtcaagaccgttctagtgaccggcggcaacggctgg ttgggtcgttggctggtgctcgattggctggagcggttggcacccaatggtggc aaggtctacgccctcattcgtggcgccgatgccgaagcagcccgggcacggttg gacgccgtgtacgaatcgggtgatcccaagctgtccgcgcattatcgtcagctg gcgcaacagagtctggaagttatcgccggcgatttcggcgaccaggatctcggt ctatcccaggaagtttggcagaagctggccaaggacgtggacctgatcgtgcac tccggtgccttggtgaaccacgtgctgccgtacagccagttgttcggtccgaat gtggcgggtaccgccgagatcatcaagctggcaatttcggagcggctcaagccg gtcacctacctgtcgacggtgggcatcgccgaccagattccggtgacggagttc gaggaagactccgatgttcgtgtgatgtcggccgagcgccagatcaatgacggc tacgcgaacggatacggcaactcaaaatgggccggcgaggtgctgttgcgggag gctcatgacctagcggggctgccggtgcgtgtgttccgctccgacatgatcctg gcgcacagtgactaccacggacagctcaacgtcaccgacgtgttcacccggagc atccagagtctgctgctcaccggtgttgcaccggccagcttctatgaattggat gccgacggcaatcggcagcgcgctcactatgacggtgtgcccggcgatttcacc gccgcatcgatcaccgccatcggcggtgtgaacgtggtagacggttaccgcagc ttcgacgtgttcaacccgcaccatgacggtgtctcgatggataccttcgtcgac tggctgatcgacgcaggctacaagatcgcgcggatcgacgattacgaccagtgg ctcgcccggttcgagctggccctcaagggattgcccgagcagcagcggcaacag tcggtgttgccacttctcaagatgtacgagaagccgcaaccggcgatcgacgga agtgcacttccgaccgcagaattcagtcgcgccgtgcacgaggcgaaggtcgga gacagcggtgagataccgcacgtcaccaaggagctgatcctcaagtacgccagc gatattcagctgttgggcctggtgtag |
| 11 | sfp | atgaagatttacggaatttatatggaccgcccgctttcacaggaagaaaatgaa cggttcatgactttcatatcacctgaaaaacgggagaaatgccggagattttat cataaagaagatgctcaccgcaccctgctgggagatgtgctcgttcgctcagtc ataagcaggcagtatcagttggacaaatccgatatccgctttagcacgcaggaa tacgggaagccgtgcatccctgatcttcccgacgctcatttcaacatttctcac tccggccgctgggtcattggtgcgtttgattcacagccgatcggcatagatatc gaaaaaacgaaaccgatcagccttgagatcgccaagcgcttcttttcaaaaaca gagtacagcgaccttttagcaaaagacaaggacgagcagacagactatttttat catctatggtcaatgaaagaaagctttatcaaacaggaaggcaaaggcttatcg cttccgcttgattccttttcagtgcgcctgcatcaggacggacaagtatccatt gagcttccggacagccattccccatgctatatcaaaacgtatgaggtcgatccc ggctacaaaatggctgtatgcgccgcacaccctgatttccccgaggatatcaca atggtctcgtacgaagagcttttataa |

**[Table 8]**

| SEQ ID NO. | Primer name | Primer sequence (5'→3') |
|---|---|---|
| 20 | Primer 1 | tgattacgccaagcttggtacgtaccttatccgcgc |
| 21 | Primer 2 | ccttcggatctaaacgatctggtgacctcttctctgaaac |
| 22 | Primer 3 | gtttcagagaagaggtcaccagatcgtttagatccgaagg |
| 23 | Primer 4 | ggcggttcttcttgttcattgtatgtcctcctggacttc |
| 24 | Primer 5 | gaagtccaggaggacatacaatgaacaagaagaaccgcca |
| 25 | Primer 6 | cgacaaacaacagataaaacgaaaggcccagtctttcgactgagcct ttcgttttatttgtcaatccgccagggcgatcg |
| 26 | Primer 7 | gactgggcctttcgttttatctgttgtttgtcggtgaacgctctcct gagtaggacaaatagatcgtttagatccgaagg |
| 27 | Primer 8 | tggtacagagcgatgcgcattgtatgtcctcctggacttc |
| 28 | Primer 9 | gaagtccaggaggacatacaatgcgcatcgctctgtacca |
| 29 | Primer 10 | ggaggggttttacttagatttcagcctttacgcaggtgca |
| 30 | Primer 11 | tgcacctgcgtaaaggctgaaatctaagtaaaacccctcc |
| 31 | Primer 12 | ccggggatcctctagaatggtgggatccatcgcg |
| 32 | Primer 13 | tgattacgccaagctagatgctcattgaccgggg |
| 33 | Primer 14 | ccttcggatctaaacgatcttgctacggcagctccaatcc |
| 34 | Primer 15 | ggattggagctgccgtagcaagatcgtttagatccgaagg |
| 35 | Primer 16 | ccaacagctttgatcttcattgtatgtcctcctggacttc |
| 36 | Primer 17 | gaagtccaggaggacatacaatgaagatcaaagctgttgg |
| 37 | Primer 18 | aattggcagctaagtagggtggttttactgctactttgat |
| 38 | Primer 19 | agtagcagtaaaaccaccctacttagctgccaattattcc |
| 39 | Primer 20 | cggtatgagagatcttccacgggtaaaaaatcctttcgta |
| 40 | Primer 21 | tacgaaaggattttttacccgtggaagatctctcataccg |
| 41 | Primer 22 | accacatagctggcttcgtcttagcccaccttctggtgcg |
| 42 | Primer 23 | cgcaccagaaggtgggctaagacgaagccagctatgtggt |
| 43 | Primer 24 | ccggggatcctctagcaagcaccggtcgattgc |
| 44 | Primer 25 | gaccatgattacgccaagctcgacgcagaaggtgtgatcc |
| 45 | Primer 26 | cgacaaacaacagataaaacgaaaggcccagtctttcgactgagcct ttcgttttatttgtttagcccaccttctggtgc |
| 46 | Primer 27 | gactgggcctttcgttttatctgttgtttgtcggtgaacgctctcct gagtaggacaaataccctacttagctgccaatt |
| 47 | Primer 28 | taaattccgtaaatcttcatgggtaaaaaatcctttcgta |
| 48 | Primer 29 | aaggattttttacccatgaagatttacggaatttatatgg |
| 49 | Primer 30 | tcacggcaaagcgaggtacttataaaagctcttcgtacg |
| 50 | Primer 31 | cgtacgaagagcttttataagtacctcgctttgccgtgac |
| 51 | Primer 32 | ggtacccggggatcctctagcgaagcttgccgtgtgcagg |
| 52 | Primer 33 | tgattacgccaagctaataagtttgcccccgatcttcaca |
| 53 | Primer 34 | aattggcagctaagtagggtgattgctgcgacagtctcat |
| 54 | Primer 35 | atgagactgtcgcagcaatcaccctacttagctgccaatt |
| 55 | Primer 36 | ggagatcgtttcagtcatgggtaaaaaatcctttcgtagg |
| 56 | Primer 37 | aaggattttttacccatgactgaaacgatctccacagcgg |
| 57 | Primer 38 | tgttcttgccacgttcctgctacaccaggcccaacagctg |
| 58 | Primer 39 | agctgttgggcctggtgtagcaggaacgtggcaagaacat |
| 59 | Primer 40 | ccggggatcctctagggcgaccagccggaaaga |

### 3-2. Construction of Corynebacterium glutamicum Expressing davB, davA, gabT, yahK, and car

The pK19ms/davBA vector constructed in Example 3-1 above was introduced into competent cells of the *Corynebacterium glutamicum* ATCC13032 strain by electroporation using an electrophorator (BIO-RAD, USA), and then the cells were plated on 2YT KM AGAR medium (containing 2YT AGAR and 15 mg/L kanamycin) and cultured in an incubator at 30°C for 2 days to obtain colonies. Among the colonies in which the first homologous recombination was induced, the colonies confirmed by PCR were cultured in 2YT liquid medium (containing 16 g/L tryptophan, 10 g/L yeast extract, and 5 g/L NaCl) for 12 hours, and then plated on 2YT sucrose AGAR medium (containing 2YT AGAR and 100 g/L sucrose), and the antibiotic marker was removed by the second homologous recombination. The selected colonies were finally analyzed by PCR and sequencing to determine whether the davB and davA genes were introduced as intended.

Thereafter, the above-described procedures were sequentially performed using the pk19ms/yahK-gabT, pk19ms/car and pk19ms/sfp vectors, thereby constructing a *Corynebacterium glutamicum* strain in which the pathway for producing 1,4-butanediol from ornithine has been established by introduction of davB, davA, gabT, yahK and car. The constructed strain was named WB01-P001.

### 3-3. Construction of Vector Expressing LAAO/MOG, gabT, yahK and car

Using the chromosomal DNA of the *Corynebacterium glutamicum* ATCC13032 strain as a template, PCR amplification was performed using primers 1 and 2, primers 3 and 41, primers 7 and 8, and primers 44 and 12. Using each of the LAAO/MOG expression vectors pKM212-LAAO/MOG, pKM212-LAAO/MOG(C254I) and pKM212-LAAO/MOG(C254L) of Example 2 as a template, PCR amplification was performed using primers 42 and 43. The resulting PCR products were amplified by crossover PCR and then inserted into the restriction enzymes HindIII and XbaI sites of the pK19mobSacB vector. The resulting vectors were named pK19ms/LAAO/MOG, pK19ms/LAAO/MOG(C254I), and pK19ms/LAAO/MOG(C254L), respectively.

Here, the Wizard Genomic DNA Purification Kit (Promega, USA) was used to extract DNA from each strain, and the primers in Table 8 above and Table 9 below were used for PCR.

Using a thermocycler (TP600, TAKARA BIO Inc., Japan) and a reaction solution containing 100 µM of each deoxynucleotide triphosphate (dATP, dCTP, dGTP, dTTP), 1 pM of oligonucleotide, and 10 ng of template DNA, PCR was performed in the presence of one unit of a PrimeSTAR Max DNA polymerase (Takara, Japan) for 25 to 30 cycles, each consisting of 30 sec at 94°C, 30 sec at 58°C, and 1 min at 72°C.

Each of the pK19ms/LAAO/MOG, pK19ms/LAAO/MOG(C254I) and pK19ms/LAAO/MOG(C254L) vectors was transformed into *E. coli* DH5a (HIT competent cells^{™}, Cat No. RH618), which was then streaked on an LB-agar plate containing 50 ug/ml of kanamycin, and cultured at 37°C for 24 hours. After the finally formed colonies were isolated and it was checked whether the inserts would be exactly present in the vector, the vector was isolated and used to produce *Corynebacterium glutamicum* with the ornithine-1,4-butanediol pathway introduced.

**[Table 9]**

| SEQ ID NO. | Primer name | Primer sequence (5'→3') |
|---|---|---|
| 60 | Primer 41 | tggcggttattcttgttcattgtatgtcctcctggacttc |
| 61 | Primer 42 | gaagtccaggaggacatacaatgaacaagaataaccgcca |
| 62 | Primer 43 | ggttttacttagattttagtccgccagcgctatttgg |
| 63 | Primer 44 | caaatagcgctggcggactaaaatctaagtaaaacccctcc |

### 3-4. Construction of Corynebacterium glutamicum Expressing LAAO/MOG, gabT, yahK and car

The pK19ms/LAAO/MOG, pK19ms/LAAO/MOG(C254I) or pK19ms/LAAO/MOG(C254L) vector constructed in Example 3-3 above was introduced into competent cells of the *Corynebacterium glutamicum* ATCC13032 strain by electroporation using an electrophorator (BIO-RAD, USA), and then the cells were plated on 2YT KM AGAR medium (containing 2YT AGAR and 15 mg/L kanamycin) and cultured in an incubator at 30°C for 2 days to obtain colonies. Among the colonies in which the first homologous recombination was induced, the colonies confirmed by PCR were cultured in 2YT liquid medium (containing 16 g/L tryptophan, 10 g/L yeast extract, and 5 g/L NaCl) for 12 hours, and then plated on 2YT sucrose AGAR medium (containing 2YT AGAR and 100 g/L sucrose), and the antibiotic marker was removed by the second homologous recombination. The selected colonies were finally analyzed by PCR and sequencing to determine whether the LAAO/MOG, LAAO/MOG(C254I) or LAAO/MOG(C254L) gene was introduced as intended.

Thereafter, the above-described procedures were sequentially performed using the pK19ms/LAAO/MOG and the pk19ms/yahK-gabT, pk19ms/car and pk19ms/sfp vectors constructed in Example 3-1 above, thereby constructing a *Corynebacterium glutamicum* strain in which the pathway for producing 1,4-butanediol from ornithine has been established by introduction of LAAO/MOG, gabT, yahK and car. The constructed strain was named WBP-0002. In addition, *Corynebacterium glutamicum* strains into which LAAO/MOG(C254I) and LAAO/MOG(C254L) mutations have been introduced using pK19ms/LAAO/MOG(C254I) and pK19ms/LAAO/MOG(C254L) were named WBP-0003 and WBP-0004, respectively.

### 3-5. Evaluation of 1,4-Butanediol Productivity

The present inventors evaluated the 1,4-butanediol productivity of each of the *Corynebacterium glutamicum* WB01-P001, WBP-0002, WBP-0003 and WBP-0004 with the ornithine-1,4-butanediol pathway introduced, constructed in Examples 3-2 and 3-4 above.

Each strain was inoculated into a flask medium (containing 5% glucose, 0.1% MgSO₄, 0.5% yeast extract, 0.2% KH₂PO₄, 1% (NH₄)₂SO₄, 20 ppm FeSO₄, 20 ppm MnSO₄, 100 µg/L biotin, 20 ppm thiamine, 20 ppm nicotinamide, and 20 ppm CPN) and cultured at 30°C for 24 hours. Here, the glucose concentration was 0 g/L. As a control group, *Corynebacterium glutamicum* ATCC13032 was used. After completion of the culturing, the culture was filtered through a 0.45 um filter and the 1,4-butanediol content in the culture was analyzed using high-performance liquid chromatography (HPLC) (Agilent, 1260 infinity II) equipped with a column (Avantor HPLC Column Apollo C18). A 0.1 M phosphate buffer was used as the mobile phase, and the analysis was performed using an RI detector at a temperature of 40°C and a flow rate of 0.8 mL/min for 15 minutes. The results are shown in Table 10 below.

**[Table 10]**

| Strain name | 1,4-butanediol (g/L) |
|---|---|
| ATCC13032 | 0 |
| WB01-P001 | 2.4 |
| WBP-0002 | 0.2 |
| WBP-0003 | 0.8 |
| WBP-0004 | 0.7 |

As shown in Table 10 above, it was confirmed that the strain WB01-P001 expressing davB and davA had significantly improved 1,4-butanediol productivity compared to the strains WBP-0002 to WBP-0004 expressing LAAO/MOG or its mutants.

So far, the present invention has been described with reference to the preferred embodiments thereof. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A microorganism that produces 1,4-butanediol from ornithine.

2. The microorganism of claim 1, wherein the microorganism has
an enzymatic reaction that converts ornithine to 4-aminobutanamide,
an enzymatic reaction that converts 4-aminobutanamide to 4-aminobutyric acid,
an enzymatic reaction that converts 4-aminobutyric acid to succinate semialdehyde,
an enzymatic reaction that converts succinate semialdehyde to 4-hydroxybutyric acid, and
an enzymatic reaction that converts 4-hydroxybutyric acid to 1,4-butanediol.

3. The microorganism of claim 2, wherein an enzyme that catalyzes the enzymatic reaction that converts ornithine to 4-aminobutanamide is lysine 2-monooxygenase.

4. The microorganism of claim 2, wherein an enzyme that catalyzes the enzymatic reaction that converts 4-aminobutanamide to 4-aminobutyric acid is 5-aminovaleramidase.

5. The microorganism of claim 2, wherein an enzyme that catalyzes the enzymatic reaction that converts 4-aminobutyric acid to succinate semialdehyde is gamma-aminobutyrate aminotransferase.

6. The microorganism of claim 2, wherein an enzyme that catalyzes the enzymatic reaction that converts succinate semialdehyde to 4-hydroxybutyric acid is alcohol dehydrogenase.

7. The microorganism of claim 2, wherein an enzyme that catalyzes the enzymatic reaction that converts 4-hydroxybutyric acid to 1,4-butanediol is alcohol dehydrogenase or carboxylic acid reductase.

8. The microorganism of any one of claims 3 to 7, wherein the enzymes are obtained by introduction of foreign genes.

9. The microorganism of any one of claims 3 to 7, wherein genes encoding the enzymes are derived from one or more microorganisms selected from the group consisting of *Escherichia coli, Pseudomonas putida, Corynebacterium glutamicum, Mycobacterium abscessus, Bacillus subtilis, Acinetobacter baumannii, Azotobacter vinelandii, Chromohalobacter salexigens, Citrobacter koseri, Citrobacter youngae, Enterobacter cloacae, Marinobacter aquaeolei, Marinomonas mediterranea, Pantoea ananatis, Pseudoalteromonas haloplanktis, Ralstonia eutropha, Shewanella putrefaciens,* and *Thiobacillus denitrificans.*

10. The microorganism of claim 1, wherein the microorganism is an *Escherichia* sp. or *Corynebacterium* sp. strain.

11. A method for producing 1,4-butanediol, comprising steps of:
culturing the microorganism of claim 1 in a medium; and
recovering 1,4-butanediol from the microorganism or the medium in which the microorganism has been cultured.
